# EUROPÄISCHE PATENTSCHRIFT

(11) **EP 1 999 264 B1**
(45) Veröffentlichungstag und Bekanntmachung des Hinweises auf die Patenterteilung: **12.05.2010**
(21) Anmeldenummer: 07727304.3
(22) Anmeldetag: 23.03.2007
(51) Int. Cl.: C12P 7/42, C12N 9/90, C12P 7/52

(54) **VERFAHREN ZUR ENZYMATISCHEN HERSTELLUNG VON 2-HYDROXY-2-METHYLCARBONSÄUREN**
METHOD FOR THE ENZYMATIC PRODUCTION OF 2-HYDROXY-2-METHYL CARBOXYLIC ACIDS
PROCEDE DE FABRICATION ENZYMATIQUE D'ACIDES 2-HYDROXY-2-METHYLCARBOXYLIQUES

(30) Priorität: 24.03.2006 DE 102006014167; 12.04.2006 DE 102006017760
(43) Veröffentlichungstag der Anmeldung: 10.12.2008
(73) Patentinhaber: Evonik Röhm GmbH, 64293 Darmstadt (DE)
(72) Erfinder: MÜLLER, Roland, H., 04425 Taucha (DE); ROHWERDER, Thore, 45472 Mühlheim an der Ruhr (DE)
(86) Internationale Anmeldenummer: PCT/EP2007/052830
(87) Internationale Veröffentlichungsnummer: WO 2007/110394

(56) Entgegenhaltungen:
- EP-A- 0 487 853
- DATABASE EMBL [Online] 15. April 2006 (2006-04-15), "Aquincola tertiaricarbonis L108 isobutyryl-CoA mutase large subunit (icmA) gene, partial cds." XP002460831 Database accession no. DQ436456 & ROHWERDER THORE ET AL: APPLIED AND ENVIRONMENTAL MICROBIOLOGY JUN 2006, Bd. 72, Nr. 6, Juni 2006 (2006-06), Seiten 4128-4135, ISSN: 0099-2240
- DATABASE EMBL [Online] 15. April 2006 (2006-04-15), "Aquincola tertiaricarbonis L108 isobutyryl-CoA mutase small subunit (icmB) gene, partial cds." XP002460834 Database accession no. DQ436457 & ROHWERDER THORE ET AL: APPLIED AND ENVIRONMENTAL MICROBIOLOGY JUN 2006, Bd. 72, Nr. 6, Juni 2006 (2006-06), Seiten 4128-4135, ISSN: 0099-2240
- DATABASE EMBL [Online] 16. Mai 2006 (2006-05-16), "Isobutyryl-CoA mutase large subunit (Fragment)" XP002460835 Database accession no. Q1PHM1 & ROHWERDER THORE ET AL: APPLIED AND ENVIRONMENTAL MICROBIOLOGY JUN 2006, Bd. 72, Nr. 6, Juni 2006 (2006-06), Seiten 4128-4135, ISSN: 0099-2240
- DATABASE EMBL [Online] 6. März 2007 (2007-03-06), "Methylmalonyl-CoA mutase (EC 5.4.99.2)" XP002460836 Database accession no. A2SP22 & KANE STACI R ET AL: "Whole-genome analysis of the methyl tert-butyl ether-degrading beta-proteobacterium Methylibium petroleiphilum PM1" JOURNAL OF BACTERIOLOGY, Bd. 189, Nr. 5, März 2007 (2007-03), Seiten 1931-1945, ISSN: 0021-9193
- DATABASE EMBL [Online] 16. Mai 2006 (2006-05-16), "Isobutyryl-CoA mutase small unit (Fragment)" XP002460837 Database accession no. Q1PHM0 & ROHWERDER THORE ET AL: APPLIED AND ENVIRONMENTAL MICROBIOLOGY JUN 2006, Bd. 72, Nr. 6, Juni 2006 (2006-06), Seiten 4128-4135, ISSN: 0099-2240
- ROHWERDER THORE ET AL: "The alkyl tert-butyl ether intermediate 2-hydroxyisobutyrate is degraded via a novel cobalamin-dependent mutase pathway." APPLIED AND ENVIRONMENTAL MICROBIOLOGY JUN 2006, Bd. 72, Nr. 6, Juni 2006 (2006-06), Seiten 4128-4135, XP002460829 ISSN: 0099-2240
- CHARLES T C ET AL: "Methylmalonyl-CoA mutase encoding gene of Sinorhizobium meliloti" GENE, ELSEVIER, AMSTERDAM, NL, Bd. 226, Nr. 1, 8. Januar 1999 (1999-01-08), Seiten 121-127, XP004154477 ISSN: 0378-1119
- RAADT DE A ET AL: "CHEMOSELECTIVE ENZYMATIC HYDROLYSIS OF ALIPHATIC AND ALICYCLIC NITRILES" JOURNAL OF THE CHEMICAL SOCIETY, PERKIN TRANSACTIONS 1, CHEMICAL SOCIETY. LETCHWORTH, GB, Bd. 1, 1992, Seiten 137-140, XP009016819 ISSN: 0300-922X
- DATABASE WPI Week 199213 Derwent Publications Ltd., London, GB; AN 1992-099664 XP002460839 & JP 04 040897 A (NITTO CHEM IND CO LTD) 12. Februar 1992 (1992-02-12)
- RATNATILLEKE ANANDA ET AL: "Cloning and sequencing of the coenzyme B12-binding domain of isobutyryl-CoA mutase from Streptomyces cinnamonensis, reconstitution of mutase activity, and characterization of the recombinant enzyme produced in Escherichia coli" JOURNAL OF BIOLOGICAL CHEMISTRY, Bd. 274, Nr. 44, 29. Oktober 1999 (1999-10-29), Seiten 31679-31685, XP002460830 ISSN: 0021-9258

## Beschreibung

Die vorliegende Erfindung betrifft ein Verfahren zur enzymatischen Herstellung von 2-Hydroxy-2-methylcarbonsäuren aus 3-Hydroxycarbonsäuren, wobei eine 3-Hydroxycarbonsäure in einer wässrigen Reaktionslösung produziert und/oder zu dieser Reaktionslösung gegeben und inkubiert wird. Die wässrige Reaktionslösung enthält eine 3-Hydroxycarbonsäure-CoA-Mutase-Aktivität aufweisende Einheit, welche sowohl 3-Hydroxycarbonyl-CoA-Ester produzierende als auch 3-Hydroxycarbonyl-CoA-Ester isomerisierende Aktivität besitzt und die Umwandlung der 3-Hydroxycarbonsäure in die entsprechende 2-Hydroxy-2-methylcarbonsäure bewirkt, welche als Säure oder in Form ihrer Salze gewonnen wird. In einer bevorzugten Ausführungsform umfasst die 3-Hydroxycarbonsäure-CoA-Mutase-Aktivität aufweisende Einheit eine isolierte cobalaminabhängige Mutase und ggf. ein 3-Hydroxycarbonyl-CoA-Ester produzierendes Enzym oder Enzymsystem oder ist ein sie enthaltender Mikroorganismus. Vorzugsweise betrifft die Erfindung einen biotechnologischen Prozess zur Produktion von 2-Hydroxy-2-methylcarbonsäuren, wobei Mikroorganismen, die die gewünschte Mutase-Aktivität besitzen, in einem wässrigen System mit Hilfe einfacher Naturstoffe kultiviert werden und intrazellulär entstehende 3-Hydroxycarbonyl-CoA-Ester zu den entsprechenden 2-Hydroxy-2-methylcarbonsäuren umgewandelt werden. Die Erfindung umfasst ebenfalls die Produktion von ungesättigten 2-Methylcarbonsäuren, wobei die gewonnenen 2-Hydroxy-2-methylcarbonsäuren durch Dehydratisierung in die korrespondierenden ungesättigten 2-Methylcarbonsäuren (Methacrylsäure und höhere Homologe) umgewandelt werden.

In einer bevorzugten Ausführung der Erfindung wird als 3-Hydroxycarbonyl-CoA-thioester produzierender und 3-Hydroxycarbonyl-CoA-thioester isomerisierender Mikroorganismus der Stamm HCM-10 (DSM 18028) eingesetzt.

Methacrylsäure sowie homologe ungesättigte 2-Methylcarbonsäuren finden breite Anwendung bei der Produktion von Acrylglasscheiben, Spritzgussformprodukten, Beschichtungen und vielen anderen Produkten.

Mehrere Prozesse zur Herstellung von Methacrylsäure und deren Homologen sind bekannt. Allerdings basiert der weltweit größte Teil der kommerziellen Produktion auf einem Verfahren zur Hydrolyse der Amid-Sulfate von Methacrylsäure und deren Homologe, die aus den entsprechenden 2-Hydroxynitrilen produziert werden (W. Bauer, "Methacrylic acid and derivatives", in: Ullmann's Encyclopedia of Industrial Chemistry, 5. Auflage, Herausgeber: B. Elvers, S. Hawkins, G. Schulz, VCH, New York, 1990, Bd. A16, S. 441-452; A. W. Gross, J. C. Dobson, "Methacrylic acid and derivatives", in Kirk-Othmer Encyclopedia of Chemical Technology, 4. Auflage, Herausgeber: J. 1. Kroschwitz, M. Howe-Grant, John Wiley & Sons, New York, 1995, Bd. 16, S. 474-506). Bei dieser Methode werden beispielsweise etwa 1,6 kg Schwefelsäure zur Produktion von 1 kg Methacrylsäure benötigt. Aus diesem Grunde wären alternative Verfahren für die kommerzielle Produktion von Methacrylsäure vorteilhaft, die ohne eine Wiedergewinnung der Schwefelsäure (und dem damit verbundenen hohen Energieaufwand) auskommt.

Die chemische Umwandlung von 2-Hydroxyisobuttersäure in Methacrylsäure ist durch die Patente US 3,666,805 and US 5,225,594 bekannt gemacht worden. Hier wird 2-Hydroxyisobuttersäure durch Einsatz von Metalloxiden, Metallhydroxiden, lonenaustauscherharzen, Tonerde, Siliziumdioxid, Amine, Phosphine, Alkalimetallalkoxide und -carboxylate dehydratisiert. Übliche Reaktionstemperaturen liegen zwischen 160 °C und 250 °C. Mit diesem Verfahren konnten Methacrylsäure-Ausbeuten von bis zu 96 % erzielt werden.

Ein alternatives Verfahren zur Produktion von Methacrylsäure und deren Homologe ergibt sich durch Hydrolyse von 2-Hydroxynitrilen zu den korrespondierenden 2-Hydroxy-2-methylcarbonsäuren unter Nutzung von Nitril-hydrolysierenden Enzymen. Dabei handelt es sich um Nitrilase oder eine Kombination aus Nitril-Hydratase und Amidase (A. Banerjee, R. Sharma, U. C. Banerjee, 2002, "The nitrile-degrading enzymes: current status and future prospects", Appl. Microbiol. Biotechnol., 60:33-44). Dieses Verfahren ist durch mehrere Patente geschützt (US 6,582,943 B1). Ein schwerwiegender Nachteil dieser Methode ist die Instabilität der Nitrile im für eine effiziente Nitril-hydrolisierende Enzymaktivität benötigten neutralen pH-Bereich. Der Zerfall der Nitrile im Reaktionsgemisch führt zu einer Akkumulation von Ketonen und Cyanid, die beide die Nitril-hydrolisierenden Enzymaktivitäten hemmen.

Ein genereller Nachteil beider Verfahren, d.h. des zurzeit dominierenden Verfahrens auf Basis der Amid-Sulfate und des enzymatischen Nitril-hydrolysierenden Verfahrens, ist der Bedarf an 2-Hydroxynitriten. Diese müssen erst aus umweltschädlichen Edukten, namentlich Ketone und Cyanid, hergestellt werden.

Deshalb wären Verfahren zur Produktion von Methacrylsäure und deren Homologe, die auf einfachen umweltunschädlichen Edukten basieren, von Vorteil.

Der Erfindung lag deshalb die Aufgabe zugrunde, nach alternativen Möglichkeiten zur Produktion von 2-Hydroxy-2-methylcarbonsäuren zu suchen und Mittel und Verfahren bereitzustellen, die möglichst auf der Anwendung einfacher, umweitunschädticher Edukte basieren, wenig Energie verbrauchen und wenig Abfallstoffe produzieren.

Die Aufgabe wird durch ein enzymatisches Verfahren zur Herstellung von 2-Hydroxy-2-methylcarbonsäuren aus 3-Hydroxycarbonsäuren gelöst. Erfindungsgemäß wird die 3-Hydroxycarbonsäure in einer wässrigen Reaktionslösung, welche eine 3-Hydroxycarbonsäure-CoA-Mutase-Aktivität aufweisende Einheit besitzt, produziert und/oder zu einer solchen Reaktionslösung gegeben. Unter einer 3-Hydroxycarbonsäure-CoA-Mutase-Aktivität aufweisenden Einheit versteht man im Sinne der Erfindung eine Einheit umfassend eine cobalaminabhängige Mutase und ggf. ein 3-Hydroxycarbonyl-CoA-Ester produzierendes Enzym oder Enzymsystem bzw. ein sie umfassendes oder produzierendes biologisches System, welche 3-Hydroxycarbonsäure-CoA-Mutase-Aktivität besitzen und sowohl 3-Hydroxycarbonyl-CoA-Ester produzierende als auch 3-Hydroxycarbonyl-CoA-Ester isomerisierende Aktivität zeigen. Nach Inkubation wird anschließend die entsprechend umgewandelte 2-Hydroxy-2-methylcarbonsäure als Säure oder in Form ihrer Salze gewonnen.

Vorzugsweise betrifft die Erfindung einen biotechnologischen Prozess zur Produktion von 2-Hydroxy-2-methylcarbonsäuren unter Verwendung von Mikroorganismen. Diese Mikroorganismen besitzen i.d.R. 3-Hydroxycarbonyl-CoA-Ester synthetisierende Aktivität und sind in der Lage, eine solche cobalaminabhängige Mutase zu produzieren bzw. umfassen eine solche Mutase und sind durch die 3-Hydroxycarbonsäure-CoA-Mutase-Aktivität in der Lage, intrazellulär aus einfachen Naturstoffen (aus Edukten, wie z.B. Zucker und/oder Alkoholen und/oder organischen Säuren und deren Derivaten) gebildete 3-Hydroxycarbonyl-CoA-Ester in die korrespondierenden 2-Hydroxy-2-methylcarbonyl-CoA-Ester umzuwandeln.

Das erfindungsgemäße Verfahren ist insbesondere **dadurch gekennzeichnet, dass** man Mikroorganismen, die die cobalaminabhängige Mutase produzieren bzw. umfassen und 3-Hydroxycarbonsäure-CoA-Mutase-Aktivität besitzen, in wässrigen Systemen zur Umwandlung von 3-Hydroxycarbonsäuren in die entsprechende 2-Hydroxy-2-methylcarbonsäure einsetzt.

In einer bevorzugten Verfahrensvariante werden Mikroorganismen, die 3-Hydroxycarbonsäure-CoA-Mutase-Aktivität umfassen und sowohl 3-Hydroxycarbonyl-CoA-Thioester produzierende als auch 3-Hydroxycarbonyl-CoA-Thioester isomerisierende Aktivität besitzen in einem wässrigen System mit nachwachsenden Rohstoffen oder aus der Verwertung nachwachsender Rohstoffe anfallender Abfallprodukte als Kohlenstoff- und Energiequelle kultiviert. Dabei werden die intrazellulär gebildeten 3-Hydroxycarbonsäure-CoA-Thioester zu den korrespondierenden 2-Hydroxy-2-methylcarbonsäuren umgewandelt. Vorzugsweise erfolgt die Reaktion unter Zugabe externer 3-Hydroxycarbonsäure. Anschließend wird die entsprechende 2-Hydroxy-2-methylcarbonsäure als Säure oder in Form ihrer Salze soliert.

Dieses neuartige biotechnologische Verfahren, das die Produktion von 3-Hydroxycarbonsäuren aus einfachen Naturstoffen und deren Isomerisierung zu 2-Hydroxy-2-methylcarbonsäuren nutzt, ist in der Lage das oben genannte Problem zu lösen.

In einer bevorzugten Ausführung der Erfindung umfasst das Verfahren die folgenden Schritte
(a) in einem geeigneten biologischen System, das 3-Hydroxycarbonyl-CoA-Ester synthetisierende Aktivität und Mutase-Aktivität besitzt, erfolgt die Produktion von 3-Hydroxycarbonsäuren aus einfachen Naturstoffen und die anschließende Umwandlung in 2-Hydroxy-2-methylcarbonsäuren und
(b) Isolierung der 2-Hydroxy-2-methylcarbonsäuren als freie Säuren oder als deren korrespondierende Salze.

Die so gewonnenen 2-Hydroxy-2-methylcarbonsäuren können vorteilhaft zur Herstellung von C2-C3-ungesättigten Isoalkensäuren (Methacrylsäure und deren Homologe) eingesetzt werden, was durch Dehydratisierung der in (a) und (b) hergestellten Säuren oder deren korrespondierenden Salzen erfolgen kann. Diese Reaktionen sind im Folgenden dargestellt:
- einfache Naturstoffe (z. B. nachwachsende Rohstoffe oder aus der Verwertung nachwachsender Rohstoffe anfallende Abfallprodukte, wie z.B. Zucker, organische Säuren oder Alkohole) → 3-Hydroxycarbonsäuren → 2-Hydroxy-2-methylcarbonsäuren (z. B. durch Stamm HCM-10)
- 2-Hydroxy-2-methylcarbonsäuren → Methacrylsäure und Homologe (z.B. in Gegenwart von NaOH und einer Temperatur von 185°C)

Die Reaktionsbedingungen (pH-Wert, lonenkonzentration, Sauerstoff/ Kohlendioxidbedarf, Spurenelemente, Temperaturen und ähnliches) werden dabei selbstverständlich so gewählt, dass die Mikroorganismen zu einer optimalen Umsetzung von 3-Hydroxycarbonsäuren zu 2-Hydroxy-2-methylcarbonsäuren befähigt sind. Unter diesen Verfahrensbedingungen kann die cobalaminabhängige Mutase im natürlichen Mikro-Milieu, also innerhalb der Zelle, eine höhere Stabilität und Effektivität aufweisen als das isolierte Enzym. Darüber hinaus kann unter geeigneten Bedingungen eine Zellvermehrung und somit eine Erhöhung der Mutase-Konzentration möglich sein. Somit bedeutet die enzymatische Umsetzung mittels Mikroorganismen ggf. einen bedeutenden Vorteil, was Zuverlässigkeit, Automatisierung und Einfachheit sowie Qualität und Ausbeute des Endproduktes des Verfahrens angeht.

Für die erfindungsgemäße enzymatische Umsetzung von 3-Hydroxycarbonsäuren in 2-Hydroxy-2-methylcarbonsäuren ist es auch möglich, die.3-Hydroxycarbonsäure-CoA-Mutase-Aktivität besitzende Einheit, also eine cobalaminabhängige Mutase vorzugsweise in Kombination mit einer CoA-Ester synthetisierenden Aktivität, in gereinigter, angereicherter und/oder isolierter Form in die Reaktionslösung einzubringen, wobei die Enzyme z.B. natürlichen Ursprungs sein können. Selbstverständlich können die Enzyme rekombinant hergestellte Enzyme aus einem gentechnisch veränderten Organismus sein.

Die Enzyme werden im erfindungsgemäßen Verfahren im Sinne der Erfindung als Katalysatoren sowohl in Form von intakten mikrobiellen Zellen als auch in Form von permeabilisierten mikrobiellen Zellen eingesetzt. Weitere Einsatzmöglichkeiten bestehen in Form von Komponenten (eine oder mehrere) aus mikrobiellen Zellextrakten, aber auch in partiell gereinigter oder gereinigter Form. Gegebenenfalls werden weitere CoA-Ester synthetisierende Enzyme, z.B. CoA-Transferase oder CoA-Synthetasen, erfindungsgemäß eingesetzt. Die enzymatischen Katalysatoren können immobilisiert sein oder an ein gelöstes oder ungelöstes Trägermaterial angeheftet sein.

In einer bevorzugten Ausführungsvariante werden bestimmte Zellkompartimente oder Teile davon voneinander getrennt oder vereinigt, das heißt, Kohlenhydratstrukturen, Lipide oder Proteine und/oder Peptide sowie Nukleinsäuren, die in der Lage sind, die Mutase-Aktivität aufweisende Einheit positiv oder negativ zu beeinflussen, können kombiniert oder getrennt werden. Um eine solche Beeinflussung bewusst zu nutzen, werden aus den Mikroorganismen z.B. fachgemäß Rohextrakte hergestellt, welche ggf. zentrifugiert werden um eine erfindungsgemäße Umsetzung mit dem Sediment oder dem Überstand durchführen zu können.

3-Hydroxycarbonsäuren (wie z. B. 3-Hydroxybuttersäure) oder genauer gesagt deren intrazellulärer CoA-Thioester 3-Hydroxycarbonyl-CoA können leicht durch eine große Zahl von Bakterienstämmen aus einfachen Naturstoffen hergestellt werden. Diese Säuren stellen die Grundbausteine/Monomere für den weit verbreiteten bakteriellen Kohlenstoff- und Energiespeicherstoff Poly-3-hydroxyalkanoat dar. Umlagerungen des Kohlenstoffs innerhalb des Skelettes von Carbonsäuren sind in bakteriellen als auch in anderen biologischen Systemen ebenfalls weit verbreitet. Bisher konnte allerdings kein biologisches System zur Umwandlung von 3-Hydroxycarbonyl-CoA-Ester in die korrespondierenden 2-Hydroxy-2-methylcarbonyl-CoA-Ester nachgewiesen werden. Die Erfindung beruht auf der überraschenden Erkenntnis, dass Systeme mit cobalaminabhängiger Mutase-Aktivität beide Eigenschaften besitzen.

Cobalaminabhängige Mutasen enthaltende Mikroorganismen sind z.B. *Methylibium petroleiphilum* PM1, *Methylibium* sp. R8 (Stammsammlung UFZ Leipzig), der βproteobakterielle Stamm HCM-10, *Xanthobacter autotrophicus* Py2, *Rhodobacter sphaeroides* (ATCC17029) oder Nocardioides sp. JS614.

Ein bevorzugt geeignetes biologisches System wurde mit dem Stamm HCM-10 gefunden. Er wurde gemäß Budapester Vertrag über die Hinterlegung von Mikroorganismen für die Zwecke von Patentverfahren bei der Deutschen Sammlung von Mikroorganismen und Zellkulturen GmbH, Braunschweig, DE unter der Nr. DSM 18028 am 13.03.2006 hinterlegt.

Unter Verwendung dieses bevorzugten biologischen Systems konnte eine besonders gute Ausbeute an 2-Hydroxy-2-methylcarbonsäuren, insbesondere von 2-Hydroxyisobuttersäure, erzielt werden. Jedoch ist die enzymatische Umsetzung durch Mikroorganismen keinesfalls auf diesen Stamm beschränkt. Sämtliche Organismen, die in der Lage sind, 3-Hydroxycarbonsäuren zu 2-Hydroxy-2-methylcarbonsäuren umzuwandeln, können erfindungsgemäß eingesetzt werden.

Dabei kann es sich um Mikroorganismen handeln, die zum einen über das gleiche Gen bzw. Genprodukt verfügen oder zum anderen ein analoges Gen besitzen, welches zu Genprodukten mit ähnlicher oder analoger Aktivität führt. D.h. 3-Hydroxycarbonyl-CoA-Mutase-Aktivitäten anderer Herkunft sind durch die Erfindung ebenfalls abgedeckt. Ebenso schließt die Erfindung transformierte Systeme, die eine oder eine ähnliche 3-Hydroxycarbonyl-CoA-Mutase-Aktivität wie Stamm HCM-10 oder die anderer Herkunft besitzen, mit ein.
Dazu können Mutanten, gentechnisch geänderte sowie isolierte Abwandlungen der Mikroorganismen zählen, z.B. Organismen, die aufgrund des Einführens einer Mutase-codierenden Nukleotidsequenz die gewünschte cobalaminabhängige Mutase-Aktivität aufweisen.

Das bevorzugt eingesetzte biologische System (Stamm HCM-10 - DSM 18028) stellt 3-Hydroxycarbonyl-CoA-Ester als Thioester, aus einfachen Naturstoffen wie Zucker und/oder organischen Säuren und/oder Alkoholen und deren Derivaten her. Im hier verwendeten bevorzugten System werden die 3-Hydroxycarbonyl-CoA-Ester durch die cobalaminabhängige Kohlenstoffskelett-umgestaltende Mutase in 2-Hydroxy-2-methylcarbonyl-CoA-Ester umgewandelt, wie in Gleichung 1 beispielhaft für den Fall des (*R*)-3-Hydroxybutyryl-CoA gezeigt ist. Der CoA-Thioester wird im System hydrolysiert und die Säure ins Kulturmedium ausgeschieden.

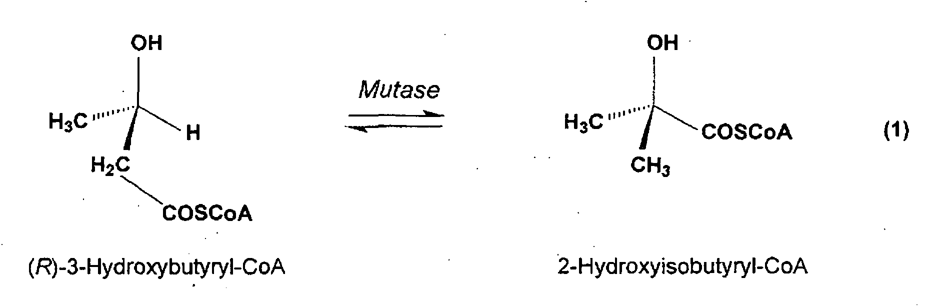

Für das erfindungsgemäße Verfahren werden als bevorzugte Enzym-Katalysatoren die cobalaminabhängige Mutasen enthaltenden Mikroorganismenstämme HCM-10 (DSM 18028), *Xanthobacter autotrophicus* Py2, *Rhodobacter sphaeroides* (ATCC17029) oder Nocardioides sp. JS614, ihre Rohextrakte oder Teile eingesetzt. Die erfindungsgemäß verwendeten Stämme produzieren bevorzugt die Proteine mit den Sequenzen SEQ ID NO: 2 und/oder SEQ ID NO: 4 bzw. umfassen die Nukleinsäuresequenzen SEQ ID NO: 1 und/oder SEQ ID NO: 3 (HCM-10), die Proteine mit den Sequenzen SEQ ID NO: 5 und/oder SEQ ID NO: 6 bzw. umfassen die Nukleinsäuresequenzen SEQ ID NO: 7 und/oder SEQ ID NO: 8 *(Xanthobacter autotrophicus* Py2), die Proteine mit den Sequenzen SEQ ID NO: 9 und/oder SEQ ID NO: 10 bzw. umfassen die Nukleinsäuresequenzen SEQ ID NO: 11 und/oder SEQ ID NO: 12 (Rhodobacter sphaeroides ATCC 17029) oder die Proteine mit den Sequenzen SEQ ID NO: 13 und/oder SEQ ID NO: 14 bzw. umfassen die Nukleinsäuresequenzen SEQ ID NO: 15 und/oder SEQ ID NO: 16 (Nocardioides sp. JS614). Im Sinne der Erfindung können die genannten Proteine auch in angereicherter, isolierter oder synthetisch hergestellter Form eingesetzt werden.

In einer weiteren bevorzugten Ausführungsvariante der Erfindung werden die Enzym-Katalysatoren, insbesondere Mikroorganismen, Rohextrakte, Teile davon und/oder die angereicherten oder isolierten Enzyme immobilisiert eingesetzt. Durch die Immobilisierung werden Enzyme, Zellorganellen und Zellen in einen unlöslichen und reaktionsraumbegrenzten Zustand versetzt. So können sie z.B. in einer Polymer-Matrix immobilisiert werden (z. B. Alginat-, Polyvinylalkohol- oder Polyacrylamid-Gele). Eine Immobilisierung kann auch auf gelösten oder ungelösten Trägermaterialien (z. B. Celit) zur Erleichterung der Katalysator-Wiedergewinnung und -benutzung erfolgen. Methoden zur Zell-Immobilisierung in einer Polymer-Matrix oder auf einem gelösten oder ungelösten Träger sind dem Fachmann bekannt und bereits ausführlich beschrieben worden. Die Enzymaktivitäten können ebenfalls aus den mikrobiellen Zellen isoliert werden. Diese können dann direkt als Katalysator oder in einer Polymer-Matrix oder auf einem gelösten oder ungelösten Träger immobilisiert eingesetzt werden. Die dazu nötigen Methoden sind dem Fachmann bekannt und beschrieben z.B. in Methods in Biotechnology, Bd. 1: Immobilization of enzymes and cells, Herausgeber: G. F. Bickerstaff, Humana Press, Totowa, New Jersey, 1997.

Die Umwandlung von 3-Hydroxycarbonsäuren in 2-Hydroxy-2-methylcarbonsäuren erfolgt bevorzugt im Rahmen eines kontinuierlichen Verfahrens, welches in einem durchströmten Reaktor durchgeführt werden kann, in dem mikrobielles Wachstum und somit die Produktbildung stattfindet. Unter einem kontinuierlichen Verfahren kann jedoch auch jedes System wachsender Zellen und katalysierender Enzyme verstanden werden, dem einerseits Nährlösung zugeführt wird und aus dem andererseits Kulturlösung, einschließlich enzymatisch gebildeter 2-Hydroxy-2-methylcarbonsäure abgezogen wird. Erfindungsgemäß kann das Verfahren auch als semikontinuierliches oder Batch-Verfahren durchgeführt werden.

Wie bereits ausgeführt wird die 3-Hydroxycarbonsäure, die der Ausgangstoff für die 2-Hydroxy-2-methylcarbonsäure ist, bevorzugt durch enzymatische Umwandlung von Kohlenhydraten und/oder organischen Säuren und/oder Alkoholen bzw. deren Derivaten hergestellt. Im Zusammenhang mit der Erfindung kommen neben der cobalaminabhängigen Mutase gegebenenfalls weiterhin CoA-Ester synthetisierende Enzyme zum Einsatz, welche im Mikroorganismus vorhanden sind oder zugegeben werden. Dabei erfolgt die Umwandlung von Kohlenwasserstoffen und/oder Kohlenhydraten und/oder organischen Säuren und/oder Alkoholen bzw. dessen Derivaten in die 3-Hydroxycarbonsäure und von der 3-Hydroxycarbonsäure in die 2-Hydroxy-2-methylcarbonsäure in einem einzigen Verfahrensschritt, d.h. die Umwandlung der Ausgangssubstrate bis hin zu der 3-Hydroxycarbonsäure und die enzymatischen Umwandlungsreaktionen der 3-Hydroxycarbonsäure in die korrespondierende 2-Hydroxy-2-methylcarbonsäure laufen gleichzeitig oder leicht zeitversetzt in ein und derselben Reaktionslösung ab.

In einer ganz besonderen Ausführung der Erfindung wird zur Kultivierung ein Substrat mit einem tert. Butylrest als Kohlenstoff- und Energiequelle eingesetzt, vorzugsweise dient tert. Butylalkohol als einzige Kohlenstoff- und Energiequelle in einem Basalmedium.

Das erfindungsgemäße Verfahren ist vorzugsweise zur Herstellung von 2-Hydroxy-2-methylpropansäure (2-Hydroxyisobuttersäure) einsetzbar. Die bevorzugte Herstellung von 2-Hydroxyisobuttersäure ist weiterhin **dadurch gekennzeichnet, dass** extern 3-Hydroxybuttersäure zugegeben wird.

Das Verfahren kann aerob, vorzugsweise beim Einsatz ganzer Zellen, durchgeführt werden oder auch anaerob, z.B. unter Stickstoffbegasung, vorzugsweise wenn Extrakte oder gereinigte Enzyme verwendet werden.

Die Erfindung betrifft auch Nukleinsäuremoleküle, codierend ein Enzym mit der Aktivität einer cobalaminabhängigen Mutase, ausgewählt aus der Gruppe bestehend aus
a) Nukleinsäuremolekülen, die ein Protein codieren, das die unter Seq. Nr. 2 und/oder Seq. Nr. 4 angegebene Aminosäuresequenzen umfasst;
b) Nukleinsäuremolekülen, die die unter Seq. Nr. 1 und/oder Seq. Nr. 3 dargestellte Nukleotidsequenz umfassen.

Es hat sich gezeigt, dass ein erfindungsgemäßes Enzym bevorzugt ein heterodimeres Protein darstellt, welches die unter Seq. Nr. 2 und Seq. Nr. 4 beschriebenen Untereinheiten umfasst und so eine hervorragende Enzymaktivität besitzt.

Ein Nukleinsäuremolekül kann ein DNA-Molekül, vorzugsweise cDNA oder genomische DNA und/oder ein RNA-Molekül sein. Sowohl Nukleinsäuren als auch Proteine können aus natürlichen Quellen isoliert werden, vorzugsweise aus DSM 18028, aber z.B. auch aus *Methylibium petroleiphilum* PM1, *Methylibium* sp. R8 (Stammsammlung UFZ Leipzig), *Xanthobacter autotrophicus* Py2, *Rhodobacter sphaeroides* (ATCC17029) oder Nocardioides sp. JS614 oder sie können nach bekannten Verfahren synthetisiert werden.

In den erfindungsgemäß verwendeten Nukleinsäuremolekülen können mittels an sich bekannter molekularbiologischer Techniken Mutationen erzeugt werden, was ermöglicht, dass weitere Enzyme mit analogen oder ähnlichen Eigenschaften synthetisiert werden können, die ebenfalls im erfindungsgemäßen Verfahren verwendet werden. Mutationen können Deletionsmutationen sein, die zu verkürzten Enzymen führen. Durch andere molekulare Mechanismen wie z.B. Insertionen, Duplikationen, Transpositionen, Genfusion, Nukleotidaustausch oder auch Gentransfer zwischen verschiedenen Mikroorganismenstämmen können ebenfalls modifizierte Enzyme mit ähnlichen oder analogen Eigenschaften erzeugt werden.

Die Identifizierung und Isolierung derartiger Nukleinsäuremoleküle kann unter Verwendung der Nukleinsäuremoleküle oder Teilern davon erfolgen. Die mit den Nukleinsäuremolekülen hybridisierenden Moleküle umfassen auch Fragmente, Derivate und allelische Varianten der oben beschriebenen Nukleinsäuremoleküle, die ein erfindungsgemäß verwendbares Enzym codieren. Unter Fragmente werden dabei Teile der Nukleinsäuremoleküle verstanden, die lang genug sind, um das beschriebene Enzym zu codieren. Unter Derivat werden Sequenzen dieser Moleküle verstanden, die sich von den Sequenzen der oben beschriebenen Nukleinsäuremoleküle an einer oder mehreren Positionen unterscheiden, aber einen hohen Grad an Homologie zu diesen Sequenzen aufweisen. Homologie bedeutet dabei eine Sequenzidentität von mindestens 40%, insbesondere eine Identität von mindestens 60%, vorzugsweise über 80% und besonders bevorzugt über 90%, 95%, 97% oder 99% auf Nukleinsäureebene. Dabei weisen die codierten Enzyme eine Sequenzidentität zu den angegebenen Aminosäuresequenzen von mindestens 60%, vorzugsweise von mindestens 80%, besonders bevorzugt von mindestens 95%, ganz besonders bevorzugt mindestens 99% auf Aminosäureebene auf. Die Abweichungen können dabei durch Deletion, Substitution, Insertion oder Rekombination entstanden sein. Es kann sich dabei um natürlich auftretende Variationen handeln, beispielsweise um Sequenzen aus anderen Organismen, oder um Mutationen, wobei diese Mutationen auf natürliche Weise auftreten können oder durch gezielte Mutagenese (UV-Strahlen, Röntgenstrahlen, chemische Mittel oder weitere). Ferner kann es sich bei den Varianten um synthetisch hergestellte Sequenzen handeln. Diese Varianten weisen bestimmte gemeinsame Charakteristika auf, wie z.B. Enzymaktivität, aktive Enzymkonzentration, Untereinheiten; funktionelle Gruppen, immunologische Reaktivität, Konformation und/oder physikalische Eigenschaften, wie das Laufverhalten in Gelelektrophorese, chromatographisches Verhalten, Löslichkeit, Sedimentationskoeffizienten, pH-Wert-Optimum, Temperatur-Optimum, spektroskopische Eigenschaften, Stabilität und/oder andere.

Gegenstand der Erfindung sind weiterhin auch die neuen Proteine mit der Sequenz Nr. 2 und 4 sowie ein heterodimeres Protein umfassend die Seq. Nr. 2 und Seq. Nr. 4 sowie die zum Protein mit der Sequenz Nr. 4 zu mindestens 99% Homologen.

SEQ ID NO: 1 zeigt die 1644 bp umfassende Nukleotidsequenz für die große Untereinheit der cobalaminabhängigen Mutase aus DSM 18028.
SEQ ID NO: 2 zeigt die 548 AS umfassende Aminosäuresequenz der großen Untereinheit der cobalaminabhängigen Mutase aus DSM 18028.
SEQ ID NO: 3 zeigt 369 bp der partiellen Nukleotidsequenz für die kleine Untereinheit der cobalaminabhängigen Mutase aus DSM 18028.
SEQ ID NO: 4 zeigt die 123 AS umfassende partielle Sequenz der Untereinheit der cobalaminabhängigen Mutase aus DSM 18028.

SEQ ID NO: 5 und 6 zeigen die 562 bzw. 135 AS umfassenden Aminosäuresequenzen einer cobalaminabhängigen Mutase aus Xanthobacter autotrophicus Py2.
SEQ ID NO: 7 und 8 zeigen die 1689 bzw. 408 bp der Nukleotidsequenz für die cobalaminabhängigen Mutasen aus Xanthobacter autotrophicus Py2.

SEQ ID NO: 9 und 10 zeigen die 563 bzw. 135 AS umfassenden Aminosäuresequenzen einer cobalaminabhängigen Mutase aus Rhodobacter sphaeroides ATCC 17029.
SEQ ID NO: 11 und 12 zeigen die 1692 bzw. 408 bp der Nukleotidsequenz für die cobalaminabhängigen Mutasen aus Rhodobacter sphaeroides ATCC 17029.

SEQ ID NO: 13 und 14 zeigen die 569 bzw. 164 AS umfassenden Aminosäuresequenzen einer cobalaminabhängigen Mutase aus Nocardoides sp. JS614.
SEQ ID NO: 15 und 16 zeigen die 1710 bzw. 495 bp der Nukleotidsequenz für die cobalaminabhängigen Mutasen aus Nocardoides sp. JS614.

Die erfindungsgemäß hergestellten 2-Hydroxy-2-methylcarbonsäuren können durch Behandlung des Kulturmediums (nach Entfernung von ungelösten Bestandteilen wie mikrobiellen Zellen) mit bereits bekannten Methoden isoliert werden. Solche Verfahren sind neben weiteren z. B. Konzentrierung, Ionenaustausch, Destillation, Elektrodialyse, Extraktion und Kristallisation. Das Produkt kann als Salz oder (nach Ansäuerung) als protonierte 2-Hydroxy-2-methylcarbonsäure isoliert werden.

2-Hydroxy-2-methylcarbonsäuren (oder deren korrespondierende Salze) können durch eine Vielzahl von Verfahren zu den entsprechenden ungesättigten 2-Methylcarbonsäuren dehydratisiert werden. Zur Herstellung von C2-C3 ungesättigten Isoalkensäuren werden die hergestellten 2-Hydroxy-2-methylcarbonsäure nach den bekannten Verfahren des Standes der Technik dehydratisiert. Die Dehydratisierung der 2-Hydroxy-2-methylcarbonsäuren kann durch Einsatz von Metalloxiden, Metallhydroxiden, lonenaustauscherharzen, Tonerde, Siliziumdioxid, Aminen, Phosphinen, Alkalimetallalkoxiden und -carboxylaten erfolgen. Übliche Reaktionstemperaturen liegen zwischen 160 °C und 250 °C. So erfolgt z.B. die Herstellung von Methacrylsäure durch Dehydratisierung der 2-Hydroxyisobuttersäure in Gegenwart von NaOH, bei Temperaturen von ca. 185°C.

Die durch diesen Prozess hergestellte Methacrylsäure und deren Homologe finden zweckdienliche Anwendung in einer ganzen Reihe von Industriezweigen, z. B. als Zusatzstoffe und in Beschichtungen. Die Methode vereinigt im Gegensatz zu den bisher bekannten Verfahren die gewünschten Vorteile eines Niedrigtemperatur-Prozesses, des Einsatzes von umweltunschädlichen Edukten und von geringer Abfallentstehung.

Anschließend wird die Erfindung an Ausführungsbeispielen näher beschrieben, auf die sie jedoch nicht beschränkt werden soll.

### Material und Methoden

### Mikrobieller Enzym-Katalysator

Mikrobielle Zellen aus Stamm HCM-10 (DSM 18028), die durch eine 3-Hydroxycarbonyl-CoA-Ester produzierende und 3-Hydroxycarbonyl-CoA-Ester isomerisierende Aktivität charakterisiert sind oder die daraus isolierten Proteinuntereinheiten mit der Sequenz Nr. 2 und Nr. 4.

### Wachstum des mikrobiellen Enzym-Katalysators

Der für die Herstellung von 2-Hydroxy-2-methylcarbonsäuren eingesetzte mikrobielle Stamm wurde wie unten beschrieben isoliert. Stammkulturen werden in 20 %iger Glyzerin-Lösung in flüssigem Stickstoff gelagert.

Stamm HCM-10 wurde aus Grundwasser auf einem Basalmedium (Tab. 1) mit tert.-Butylalkohol als alleiniger Kohlenstoff- und Energiequelle angereichert. Phylogenetisch gehört der Stamm zur Rubrivivax-Leptothrix-Gruppe.

**Tabelle 1**

| Basalmedium (mg/L) | | | |
|---|---|---|---|
| NH₄C1 | 761,4 | Biotin | 0,02 |
| KH₂PO₄ | 340,25 | Folsäure | 0,02 |
| K₂HPO₄ | 435,45 | Pyridoxin-HCl | 0,1 |
| CaCl₂ x 6 H₂O | 5,47 | Thiamin-HCl | 0,05 |
| MgSO₄ x 7 H₂O | 71,2 | Riboflavin | 0,05 |
| ZnSO₄ x 7 H₂O | 0,44 | Nicotinsäure | 0,05 |
| MnSO₄ x H₂O | 0,615 | DL-Ca-Pantothenat | 0,05 |
| CuSO₄ x 5 H₂O | 0,785 | p-Aminobenzoesäure | 0,05 |
| CoCl₂ x 6 H₂O | 0,2 | Liponsäure | 0,05 |
| Na₂MoO₄ x 2 H₂O | 0,252 | | |
| FeSO₄ x 7 H₂O | 4,98 | pH | 7,0 |

Stamm HCM-1 0 wurde aerob unter den folgenden Bedingungen (Tab. 2) zum Testen der 3-Hydroxycarbonyl-CoA-Mutase-Aktivität angezogen.

**Tabelle 2**

| Stamm | Substrat | Medium | Temperatur (°C) | Dauer (d) |
|---|---|---|---|---|
| HCM-10 | tert.-Butylalkohol (0,5 g/L) | Basalmedium | 25 | 7 |

Zellen wurden unmittelbar nach der Ernte eingesetzt. Intakte Zellen können ohne weitere Vorbehandlung, wie z. B. Permeabilisierung, eingesetzt werden. Außerdem können die Zellen permeabilisiert verwendet werden (z. B. durch Behandlung mit Toluen, Detergenzien oder durch Gefrier-Auftau-Zyklen), um die Diffusionsraten von Stoffen in die Zellen hinein und aus den Zellen hinaus zu verbessern.

Die Konzentration der 2-Hydroxyisobuttersäure und 3-Hydroxybuttersäure in der Kulturflüssigkeit bzw. im Reaktionsansatz wurden durch Gaschromatographie nach saurer Methanolyse unter Nutzung einer FFAP Säule und eines FID Detektors bestimmt.

### Beispiel 1:

### Konversion von 3-Hydroxybuttersäure in 2-Hydroxyisobuttersäure durch Stamm HCM-10

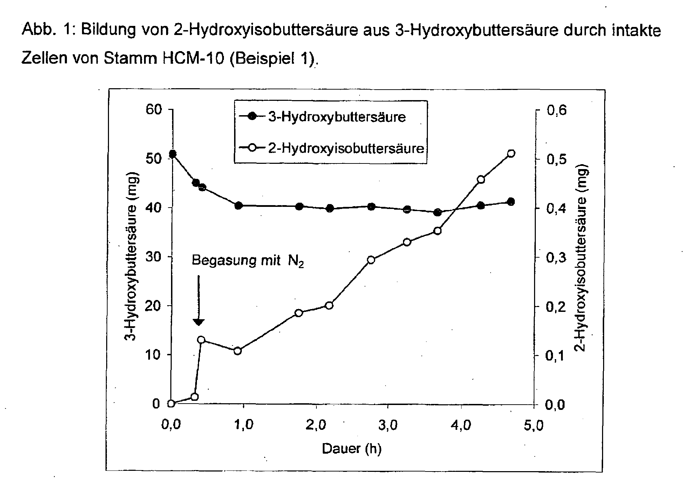

Eine Suspension von 1 g (Trockengewicht) Zellen von Stamm HCM-10 in 100 mL Basalmedium wurde in 120-mL-Serumflaschen gefüllt. Zu dieser Suspension wurden 50 mg 3-Hydroxybuttersäure gegeben und die Suspension auf einem rotierenden Schüttler bei 30°C inkubiert. Nach 0,3 h aerober Inkubation wurde die Suspension mit Stickstoff begast und weitere 4,4 h bei 30°C unter Schütteln inkubiert. Zu verschiedenen Zeiten wurden Proben genommen und der Gehalt an 2-Hydroxyisobuttersäure und 3-Hydroxybuttersäure im zellfreien Überstand nach Zentrifugation der Suspension bestimmt. 2-Hydroxyisobuttersäure wurde als alleiniges Produkt festgestellt, das in der anaeroben Phase freigesetzt wurde. In der aeroben initialen Phase wurde 3-Hydroxybuttersäure dagegen offensichtlich komplett abgebaut (Abb. 1). Die Ausbeute an 2-Hydroxyisobuttersäure war in diesem Fall 5,1 %, ca. 80 % der 3-Hydroxybuttersäure verblieben in der Reaktionsflüssigkeit.

### Beispiel 2:

### Konversion von 3-Hydroxybuttersäure in 2-Hydroxyisobuttersäure durch Rohextrakt von Stamm HCM-10

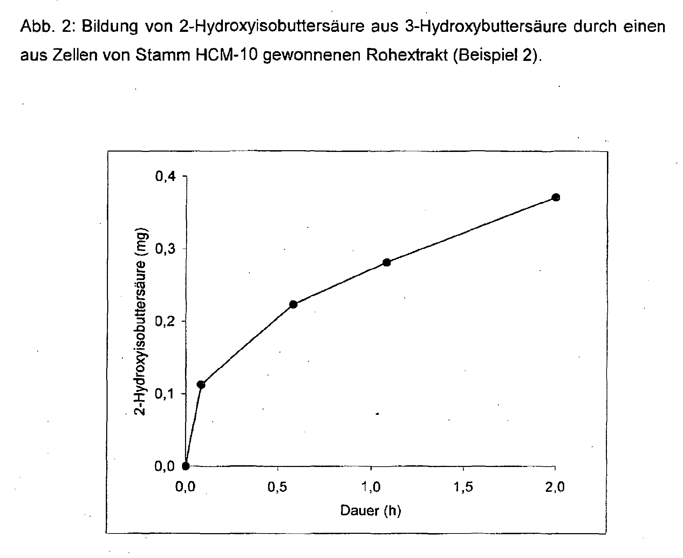

Zellfreier Rohextrakt von Stamm HCM-10 wurde durch Desintegration der Zellen in einer Kugelmühle hergestellt, Zellbruchstücke wurden nachfolgend durch Zentrifugation abgetrennt. Zellfreier Rohextrakt in einer Konzentration von 10 mg Protein in 5 mL 50-mM-Kaliumphosphatpuffer (enthält 1 mM MgCl₂ bei pH 7,2) wurde in verschließbare 10-mL-Glasgefäße gegeben. Diesem Extrakt wurde im Weiteren 0,01 mM Coenzym B12, 1 mM CoenzymA, 1 mM ATP und 4,25 mg 3-Hydroxybuttersäure zugesetzt. Die Reaktionsflüssigkeit wurde mit Stickstoff begast, das Reaktionsgefäß dicht verschlossen und unter Schütteln 2 h bei 30°C inkubiert. Die Reaktionsprodukte wurden wie oben gezeigt analysiert. Die Ausbeute an 2-Hydroxyisobuttersäure war in diesem Fall 9 %, ca. 88 % der 3-Hydroxybuttersäure verblieben in der Reaktionsflüssigkeit (Abb. 2).

### Beispiel 3:

### Dehydratisierung von 2-Hydroxyisobuttersäure zu Methacrylat

Eine Lösung von 2-Hydroxyisobuttersäure (1 mg/5 mL), produziert entspr. der in Beispiel 2 ausgeführten Prozedur wurde unter Rühren mit NaOH (0.06 mg) versetzt. Die Lösung wurde unter Rührung und Rückflusskühlung bei 185-195°C unter Vakuum (300 torr) inkubiert. Über einen Zeitraum von 5 h wurden stündlich weitere Aliquote von 0.5 mg 2-Hydroxyisobuttersäure pro 5 mL zugesetzt, diese enthielten zusätzlich 0.4 Gewichtsprozent p-Methoxyphenol, um ein Polymerisieren von Methacrylat zu verhindern. Nach 24 h Inkubation wurde die Reaktion beendet. Der Umsatz an 2-Hydroxyisobuttersäure zu Methacrylat belief sich auf 97%. Die Abtrennung von Methacrylsäure aus dem Reaktionsansatz erfolgte durch Destillation.

### Anhang Sequenzen

Sequenz Nr. 1 - 1644 bp (HCM-10; DSM 18028) 
AAGTCCCAACTCCAATCGGAGCGCAAGGACTGGGAAGCGAACGAAGTCGGCGCCTTCTTGAAGA
AGGCCCCCGAGCGCAAGGAGCAGTTCCACACGATCGGGGACTTCCCGGTCCAGCGCACCTACA
CCGCTGCCGACATCGCCGACACGCCGCTGGAGGACATCGGTCTTCCGGGGCGCTACCCGTTCA
CGCGCGGGCCCTACCCGACGATGTACCGCAGCCGCACCTGGACGATGCGCCAGATCGCCGGCT
TCGGCACCGGCGAGGACACCAACAAGCGCTTCAAGTATCTGATCGCGCAGGGCCAGACCGGCA
TCTCCACCGACTTCGACATGCCCACGCTGATGGGCTACGACTCCGACCACCCGATGAGCGACGG
CGAGGTCGGCCGCGAGGGCGTGGCGATCGACACGCTGGCCGACATGGAGGCGCTGCTGGCCG
ACATCGACCTCGAGAAGATCTCGGTCTCGTTCACGATCAACCCGAGCGCCTGGATCCTGCTCGC
GATGTACGTGGCGCTCGGCGAGAAGCGCGGCTACGACCTGAACAAGCTGTCGGGCACGGTGCA
GGCCGACATCCTGAAGGAGTACATGGCGCAGAAGGAGTACATCTACCCGATCGCGCCGTCGGT
GCGCATCGTGCGCGACATCATCACCTACAGCGCGAAGAACCTGAAGCGCTACAACCCGATCAAC
ATCTCGGGCTACCACATCAGCGAGGCCGGCTCCTCGCCGCTCCAGGAGGCGGCCTTCACGCTG
GCCAACCTGATCACCTACGTGAACGAGGTGACGAAGACCGGTATGCACGTCGACGAATTCGCGC
CGCGGTTGGCCTTCTTCTTCGTGTCGCAAGGTGACTTCTTCGAGGAGGTCGCGAAGTTCCGCGC
CCTGCGCCGCTGCTACGCGAAGATCATGAAGGAGCGCTTCGGTGCAAGAAATCCGGAATCGATG
CGGTTGCGCTTCCACTGTCAGACCGCGGCGGCGACGCTGACCAAGCCGCAGTACATGGTCAAC
GTCGTGCGTACGTCGCTGCAGGCGCTGTCGGCCGTGCTCGGCGGCGCGCAGTCGCTGCACACC
AACGGCTACGACGAAGCCTTCGCGATCCCGACCGAGGATGCGATGAAGATGGCGCTGCGCACG
CAGCAGATCATTGCCGAGGAGAGTGGTGTCGCCGACGTGATCGACCCGCTGGGTGGCAGCTAC
TACGTCGAGGCGCTGACCACCGAGTACGAGAAGAAGATCTTCGAGATCCTCGAGGAAGTCGAGA
AGCGCGGTGGCACCATCAAGCTGATCGAGCAGGGCTGGTTCCAGAAGCAGATTGCGGACTTCG
CTTACGAGACCGCGCTGCGCAAGCAGTCCGGCCAGAAGCCGGTGATCGGGGTGAACCGCTTCG
TCGAGAACGAAGAGGACGTCAAGATCGAGATCCACCCGTACGACAACACGACGGCCGAACGCC
AGATTTCCCGCACGCGCCGCGTTCGCGCCGAGCGCGACGAGGCCAAGGTGCAAGCGATGCTCG
ACCAACTGGTGGCTGTCGCCAAGGACGAGTCCCAGAACCTGATGCCGCTGACCATCGAACTGGT
GAAGGCCGGCGCAACGATGGGGGACATCGTCGAGAAGCTGAAGGGGATCTGGGGTACCTAC
Sequenz Nr. 2 - 548 aa (HCM-10; DSM 18028) 
KSQLQSERKDWEANEVGAFLKKAPERKEQFHTIGDFPVQRTYTAADIA
DTPLEDIGLPGRYPFTRGPYPTMYRSRTWTMRQIAGFGTGEDTNKRF
KYLIAQGQTGISTDFDMPTLMGYDSDHPMSDGEVGREGVAIDTLADME
ALLADIDLEKISVSFTINPSAWILLAMYVALGEKRGYDLNKLSGTVQADI
LKEYMAQKEYIYPIAPSVRIVRDIITYSAKNLKRYNPINISGYHISEAGS
SPLQEAAFTLANLITYVNEVTKTGMHVDEFAPRLAFFFVSQGDFFEEV
AKFRALRRCYAKIMKERFGARNPESMRLRFHCQTAAATLTKPQYMVN
VVRTSLQALSAVLGGAQSLHTNGYDEAFAIPTEDAMKMALRTQQIIAE
ESGVADVIDPLGGSYYVEALTTEYEKKIFEILEEVEKRGGTIKLIEQGW
FQKQIADFAYETALRKQSGQKPVIGVNRFVENEEDVKIEIHPYDNTTAE
RQISRTRRVRAERDEAKVQAMLDQLVAVAKDESQNLMPLTIELVKAGA
TMGDIVEKLKGIWGTY
Sequenz Nr. 3 - 370 bp (HCM-10; DSM 18028) 
GTTCTTCTCGCCAAAGTCGGCCTCGACGGCCATGACCGAGGCGTCAAGGTCGTCGCTCGCGCG
CTGCGCGACGCCGGCATGGACGTCATCTACTCCGGCCTTCATCGCACGCCCGAAGAGGTGGTC
AACACCGCCATCCAGGAAGACGTGGACGTGCTGGGTGTAAGCCTCCTGTCCGGCGTGCAGCTC
ACGGTCTTCCCCAAGATCTTCAAGCTCCTGGACGAGAGAGGCGCTGGCGACTTGATCGTGATCG
CCGGTGGCGTGATGCCGGACGAGGACGCCGCGGCCATCCGCAAGCTCGGCGTGCGCGAGGTG
CTACTGCAGGACACGCCCCCGCAGGCCATCATCGACTCGATCCGCTCCTTGGTCG
Sequenz Nr. 4 -123 aa (HCM-10; DSM 18028) 
VLLAKVGLDGHDRGVKVVARALRDAGMDVIYSGLHRTPEEVVNTAIQE
DVDVLGVSLLSGVQLTVFPKIFKLLDERGAGDLIVIAGGVMPDEDAAAI
RKLGVREVLLQDTPPQAIIDSIRSLV
Sequenz Nr. 7 - 1689 bp DNA, Xanthobacter autotrophicus Py2
   NZU_AAPC01000001.1:231146..232834 (mit Sequenz 1 von HCM-10: Identität = 1283/1598 (80%), Gaps = 0/1598 (0%)) 
1 tcagaacacc ggtgtttcgc gatacacgcc ccacaccgtc ttcagccgct ccacgatgtc
61 gcccatggtc gcgccttcac gcacgagctc gatggtgaca ggcatgatgt tctgggtctc
121 gtccttcgcc acctccacca ggcggtcgag cagcgcgctg accttcacat tgtcgcgctc
181 acggcggacc cgctgggtgc gggcgatctg gcggtcggcg gtggtgtggt cataagggtg
241 caattcgatc tcatgcacct cgctttcgtc caccatggtg tttacgccga tcaccggctt
301 ttcgcccgac tgcttgcgca gcgccgtctc gtaggcgaag tcggcgacgc tcttctggaa
361 ccagccttct tgaatcagct tgatggtgcc accgcgcgcg tccacctcgg agatgatgtc
421 catgatgcgc ttttcatact cggtggtgag cgtctccaca taataggagc cccccacggg
481 atcgatcacc tgggtgacgt tgctttcttc ggcgatcacc tgctgggtgc gcagggcaag
541 gcgcatggcc tcctcggtgg ggatggcgaa cgcctcgtcg aagccgttgg tatgcagcga
601 ctggcagccg cccagcaccg ccgccagcgc ctgcagcgtc gtgcgcacca cgttcaccat
661 gaattgcggc ttggtgagcg aggcggcggc ggtctggcag tggaagcgca ggcgcatgga
721 ttccgggttc ctggccccga agcgctcctt catgatcttg gcgtagcagc gccgcacggc
781 gcggaacttg gcgatctcct cgaagaagtc ggcctggcag acgaagaaga aggcgaggcg
841 cggggcgaag tcatccactt tcatgccggt cttcaacacc tcctccacat agacgatgag
901 gttcgccagg gtgaaggcca cctcgtccac cggagaggag ccggcttcgg agatatggta
961 gccggaaatg ttgatggggt tgtagcgctt catgttcttg gcgcagtacg tgatgcagtc
1021 gcgcacgatg cgcacggaag gcgcgatggg atagatatat tccttctgcg ccatgtactc
1081 cttcaggatg tccgcttgga cggtgccgga caatttgtcg aggtcgtagc cgcgcttctg
1141 cgccagcacg atatacatgg cgagcaggat ccacgcggac ggattgatgg tcatcgagac
1201 cgagatcttc tcgagatcga tcccgtcgaa cagcgcctcc atgtcggcga gcgtgtcgat
1261 ggccacgccc tcgcggccca cctcgccgtc gctcatcgga tggtcgctgt cgtaacccat
1321 gagggtcggc atgtcgaagt cggtggagat gccggtctgc ccctgttcga tcaggtattt
1381 gaagcgcttg ttcgtgtcct cgccggtccc gaagccggcg atctggcgca tggtccagtt
1441 ccggctgcgg tacatggtgg gataggggcc gcgggtgaag gggtagcggc cgggcagccc
1501 gatgtcctcg atcggcgtcg ccgccgcgtc ggcggcggtg tagacgcgct tcaccgggaa
1561 gtcgccgagg gtgaagaact gctccttgcg ctccttctgc ttcttcagga aggcggcgac
1621 ctcggtctcc tcccagtgac gcacgtcgtc gtcgagattg gcgagggtca tgatctcaga
1681 catgctcat
Sequenz Nr. 5 - 562 aa, Xanthobacter autotrophicus Py2
   ZP_01195960; (mit Sequenz 2 von HCM-10: Identität = 445/547 (81%), Positives = 492/547 (89%), Gaps = 0/547 (0%)) 
msmseimtla nldddvrhwe etevaaflkk qkerkeqfft lgdfpvkrvy taadaaatpi
ediglpgryp ftrgpyptmy rsrnwtmrqi agfgtgedtn krfkylieqg qtgistdfdm
ptlmgydsdh pmsdgevgre gvaidtladm ealfdgidle kisvsmtinp sawillamyi
vlaqkrgydl dklsgtvqad ilkeymaqke yiypiapsvr ivrdcityca knmkrynpin
isgyhiseag sspvdevaft lanlivyvee vlktgmkvdd faprlafffv cqadffeeia
kfravrrcya kimkerfgar npesmrlrfh cqtaaasltk pqfmvnvvrt tlqalaavlg
gcqslhtngf deafaiptee amrlalrtqq viaeesnvtq vidpvggsyy vetltteyek
rimdiisevd arggtikliq egwfqksvad fayetalrkq sgekpvigvn tmvdesevhe
ielhpydhtt adrqiartqr vrrerdnvkv salldrlvev akdetqnimp vtielvrega
tmgdiverlk tvwgvyretp vf
Sequenz Nr. 8; 408bp DNA, Xanthobacter autotrophicus Py2
   NZ_AAPC01000001.1:235266..235673; (mit Sequenz 3 von HCM-10: Identität = 226/274 (82%), Gaps = 0/274 (0%)) 
1 tcaacgaggt ccgcgctcgg cgaccaggcg ctcgagcgtt tccacgatgg ccttgggcgg
61 ggtgtcctgg agcatcacct ccttcacacc cagttccttc agagcgacca cgtcctcgtc
121 cggcatcacg ccgccggcga tcacgatgag gtcgtccgcg ccccgctcgg cgagcagctt
181 gaagatcttg ggaaagaccg tgagctggac tccggagagg agggagacgc cgaggatgtc
241 cacgtcctcc tgcaccgccg cggtcacaac ttcctcgggc gtgcggtgga ggccggaata
301 aatcacgtcc atgccggcgt cgcgcagggt gcgcgccacc accttcacgc cgcgatcgtg
361 accgtcgagc ccgactttgg cgagcagcac cctgatcggc tgagccat
Sequenz Nr. 6, 135 aa, Xanthobacter autotrophicus Py2
   ZP_01195963; (mit Sequenz 4 von HCM-10: Identität = 102/123 (82%), Positives = 114/123 (92%), Gaps = 0/123(0%)) 
maqpirvlla kvgldghdrg vkvvartlrd agmdviysgl hrtpeevvta avqedvdilg
vsllsgvqlt vfpkifklla ergaddlivi aggvmpdedv valkelgvke vmlqdtppka
ivetlerlva ergpr
Sequenz Nr. 11, 1692 bp DNA, Rhodobacter sphaeroides ATCC 17029
   NZ_AAMF01000011.1:59715..61406; (mit Sequenz 1 von HCM-10: Identität = 1302/1568 (83%), Gaps = 011568 (0%)) 
1 tcagaacacc ggcgtttcgc ggtagacgcc ccagatcgtc ttcagcttct cgacgatgtc
61 gcccatggtc gccccctcct tcacgagctg gatggtcacg ggcatcacgt tggcgctctc
121 gtccctggcg accgcaacga gttcgtcgag gagcgcctga accttggcct cgtcgcggcc
181 cgcgcgcacc gagcgcgtgc ggtcgatctg ccgcttggcg gtcgtctcgt cgtagggatg
241 gacctcgatc ttcacatggc tctcgtccat cacatagcgg ttcacgccga tcaccggctt
301 ctcgcccgac tgcttgcgca gcgccgtctc gtaggcgaag tcggcgatgt gcttctggaa
361 ccagccttcc tcgatcagct tgatcgcccc gccacgctct tccacctcgt cgaggatctc
421 gaagatcttc ttctcgtagt cgttggtcag gctctcgacg aaataggacc cgccaagggg
481 atcgaccacc tgcgtcaccc cggtctcgtc ggcgatcacc tgctgggtgc gcagcgcgag
541 ctgcatcgca tgttcggtcg ggatggcgaa ggcctcgtca tagccgttcg tatggagcga
601 ctgcgcgccc cccagcaccg ccgccagcgc ctgggtcgtc gtgcgcatga cattgaccat
661 gtactgcggc ttggtgagcg aggccgccgc ggtctggcag tgaaagcgca ggcgcatcga
721 ctcgggtttc ttcgcgccga accgctcctt catgatcttg gcatagcagc ggcggagcgc
781 ccggaacttc gcgatctcct cgaagaagtc ggcctgacag acgaagaaga aggcgagccg
841 cggggcgaag tcgtcgacct cgacgccggt cttcagcacc tcctcgacat agacgatcag
901 gttggccagc gtgaaggcgg cctcgtgtag cggcgacgag cccgcttccg agatgtggta
961 gcccgagatg ttgatcgggt tgtagcgctt catcgtccgg gtcgagtggg agatgatgtc
1021 gcgcacgatg cggaccgagg gcgcgatcgg gaagatatat tccttctgcg ccatatattc
1081 cttgaggatg tcggcctgca ccgtgccgga caccttgttc aggtcgtagc cccgctcctc
1141 gcagagcgcg atatacatcg cgagcaggat ccaggcggtg ggattgatgg tcagcgagac
1201 cgagatcttc tcgagatcga tcccgtcgag cagcgcgcgc atgtcggcca gcgtgtcgat
1261 ggccacgccc tcgcggccca cctcgccgtc cgacatcgga tggtcgctgt cgtagcccat
1321 cagcgtcggc atgtcgaaat cggtcgagat gccggtctgg ccctgctcga tcaggaactt
1381 gaaccgcttg ttggtgtcct cgccggtgcc gaagcccgcg atctggcgca tggtccagtt
1441 gcggccgcga tacatggtcg gatagggccc gcgcgtgaag ggatatttgc ccggcaggcc
1501 gatatcctcg agcggcgtgt cggcgatgtc ggcggccgtg taggtccgct tcaccgggaa
1561 gtcgcccagc gtgtagaact gctccttgcg ctcgggcgcg cgggcgatga agtcggcgac
1621 ctcggtgcgt tcccagtcct cgaccgcttg tgcgatctcg tcggtgcggg cgtgcgagaa
1681 ggtgtcagac at
Sequenz Nr. 9, 563 aa, Rhodobacter sphaeroides ATCC 17029
   ZP_00919811; (mit Sequenz 2 von HCM-10: Identität = 451/540 (83%), Positives = 499/540 (92%), Gaps = 0/540 (0%)) 
1 msdtfshart deiaqavedw ertevadfia raperkeqfy tlgdfpvkrt ytaadiadtp
61 ledigIpgky pftrgpyptm yrgrnwtmrq iagfgtgedt nkrfkflieq gqtgistdfd
121 mptlmgydsd hpmsdgevgr egvaidtlad mralldgidl ekisvsltin ptawillamy
181 ialceergyd lnkvsgtvqa dilkeymaqk eyifpiapsv rivrdiishs trtmkrynpi
241 nisgyhisea gssplheaaf tlanlivyve evlktgvevd dfaprlafff vcqadffeei
301 akfralrrcy akimkerfga kkpesmrlrf hcqtaaaslt kpqymvnvmr tttqalaavl
361 ggaqslhtng ydeafaipte hamqlalrtq qviadetgvt qvvdplggsy fvesltndye
421 kkifeildev eerggaikli eegwfqkhia dfayetalrk qsgekpvigv nryvmdeshv
481 kievhpydet takrqidrtr svragrdeak vqalldelva vardesanvm pvtiqlvkeg
541 atmgdivekl ktiwgvyret pvf
Sequenz Nr. 12, 408 bp DNA, Rhodobacter sphaeroides ATCC 17029
   NZ_AAMF01000011.1:63815..64222; (mit Sequenz 3 von HCM-10: Identität = 259/345 (75%), Gaps = 0/345 (0%)) 1 tcagcggtct ccccgcgcct tcaccagacc cgtgagggtg tcgatgatgt gctggggcgg 
61 cgtgtcctgc agcagcacct cggccacgcc catcttcttc agctcgatca catcctcgtc
121 gggcatgacg ccccccgcga ccacgatcat gtcctcgacc ttgtgctcct tcagcagcgc
181 gaagatcttc ggaaagaccg tcatctgcac gcccgacagg aggctgacgc cgaggatgtc
241 cacatcctcc tgcaccgcgg ccgtcaccac ctcctccggg gtgcggtgga gaccggaata
301 gacgacatcc atcccggcat cgcgcagggt gcgggccacc accttcacac cgcggtcgtg
361 gccatcgagg ccaaccttcg cgagcagcac acggatctgg gtcgacat
Sequenz Nr. 10, 135 aa, Rhodobacter sphaeroides ATCC 17029
   ZP_00919814, (mit Sequenz 4 von HCM-10: Identität = 96/123 (78%), Positives = 107/123 (86%), Gaps = 0/123 (0%)) 
1 mstqirvlla kvgldghdrg vkvvartlrd agmdvvysgl hrtpeevvta avqedvdilg
61 vsllsgvqmt vfpkifallk ehkvedmivv aggvmpdedv ielkkmgvae vllqdtppqh
121 iidtltglvk argdr
Sequenz Nr. 15, 1710 bp DNA, Nocardioides sp. JS614
   NZ_AAJB01000007.1:74197..75906; (mit Sequenz 1 von HCM-10: Identität = 775/1059 (73%), Gaps =0/1059 (0%)) 
1 tcagtacaca ggtgtctcga cgtaggttcc gaacacgtcg cgtagtgcac cggtgatctc
61 gcccatcgac agaccggctc ggacagcggc gatggttgcc ggcatgagat tggcgtcgtt
121 gtcgcgagct gtttcgacca gggttgcgag agctgcctgt gcggtctcgg cgttgcgctc
181 cgcacgtacc tgctgcacgc ggcggatctg gcgctgctcg gactcggggt cgagctggtg
241 tgtctcgatc tcttccgtcg agttctcatc gatgtacata ttgaccccaa gcacagggcg
301 gtcgccgctc tccttgcgga gcgcgtactg gtaggccgtg tctgagatct cgcgctgcat
361 aaagccgttc tcgatcgctg ctacgactcc accaaggctt tcgatcttgg ctagatagtc
421 gtgaattccc gtttcgatct catcggtaag tgcctcgacg tagtacgagc cgccgagggg
481 gtcgacaatg ctaggaacgc cagtctcatg tgcgatgacc tgctgggtgc gaagcgcgat
541 cttcatggcg gtttcgctcg ggatcgtgta cgcctcgtcg agaccgttgg tgtggagcga
601 ctgtgccccg cccaggattg ctgagagggc ttggagcgtc gtgcggatga tgttgttcat
661 gggttgtggc ttggtcagcg ttgccgctgc agtctgagcg tggaaccgga gccgcatgga
721 ctgttcgttc tcggctccga actcgtcgcg catcatcttg gcgtagaagc gccggacagc
781 cctgaacttt gcggcttcct cgaagaagtc agcctggctc acgaagaaga acgacagtcg
841 cggagcgaac gtgtcgacgt cgacgccggc cgcaatgacg tcactcacgt aggcgcgcgt
901 gatcgccatg gtgaaggcga cctcctgcac ggcgttcgcg ccggcttcgc tgatgtggta
961 cccgctgatg ttgaccgggt tataacgggc catattctca gcgcaataag cgatgcagtc
1021 tcggacaatg cgcatactcg gacgaaccgg gaagatccat tccttttggg cgacgtactc
1081 cttgaggatg tcgttctgaa tcgttccgct cagtcggttc aggtcgtagc ccttgtcttc
1141 ggctacagcg atgtacatgg ccagcaggat ccaggcagac gggttgatgg tcatcgacac
1201 ggagatgttc tccaggtcga ttccgtcgaa gagtgcctcc atgtcggaga gcacgtcgac
1261 ggcgactcct tcgcggccga cctctcccag actcatgggg tcgtcgctgt cgagacccat
1321 cagtgtgggc atgtcgaagt cgacggagag gcccgtctgt ccctggttga tgaggtactg
1381 gaagcgcttg ttggtctctt cggcttgacc gaatccggcg atctggcgca tcgtccagtg
1441 gcggccccgg tacatggtcg ggtaaggccc tcgcgtgaac gggaattgac ccggaagtcc
1501 gatatcgttc cagtcctcgg gaaggtcagc cggcgtgtag accctcttga cgggggcgcc
1561 gctgccagtg cggaacactc ccttcgactc tggcgtccgt tgcaagaatg cctcgagctc
1621 gtggctctcc caccctgcga gtcgttcctc gatggccttg acggcggcct cgttggtgat
1681 cttgatggcg tcggcctcgt gggtgctcat
Sequenz Nr. 13, 569 aa, Nocardioides sp. JS614
   ZP_00656557; (mit Sequenz 2 von HCM-10: Identität = 341/538 (63%), Positives = 421/538 (78%), Gaps = 1/538 (0%)) 
1 mstheadaik itneaavkai eerlagwesh eleaflqrtp eskgvfrtgs gapvkrvytp
61 adlpedwndi glpgqfpftr gpyptmyrgr hwtmrqiagf gqaeetnkrf qylinqgqtg
121 lsvdfdmptl mgldsddpms lgevgregva vdvlsdmeal fdgidlenis vsmtinpsaw
181 illamyiava edkgydlnrl sgtiqndilk eyvaqkewif pvrpsmrivr dciaycaenm
241 arynpvnisg yhiseagana vqevaftmai trayvsdvia agvdvdtfap rlsfffvsqa
301 dffeeaakfr avrrfyakmm rdefgaeneq smrlrfhaqt aaatltkpqp mnniirttlq
 
361 alsailggaq slhtngldea ytipsetamk ialrtqqvia hetgvpsivd plggsyyvea
421 ltdeietgih dylakieslg gvvaaiengf mqreisdtay qyalrkesgd rpvlgvnmyi
481 densteeiet hqldpeseqr qirrvqqvra emaetaqaa latlvetard ndanlmpati
541 aavraglsmg eitgalrdvf gtyvetpvy
Sequenz Nr. 16, 495 bp DNA, Nocardioides sp. JS614
   NZ_AAJB01000007.1:78317..78811: (mit Sequenz 3 von HCM-10: Identität = 138/173 (79%), Gaps = 0173 (0%)) 
1 tcacgaggcc tctacctggt tccgggcctg gttcgcggcg tcggtgacga cctcgacgat
61 gtggcgcggc gcggtgtcct ggccgaggac agcggcgaca cccagttcga tcagggtcct
121 ctcgtcctcg tcgggcatga cgccgccagc gacgacggca aatcgtggcg tgtagggaag
181 atctgcgatg agcgtgaaga gtcgaccgaa aatggtcagg tgtgctccgg agagcaggct
241 gatgccgagc acgtcgacgt cttcttgcac ggcggcttcg aggacctgct ctgggctgcg
301 gtgaaggccg gtgtagacga cctccatgcc ggcgtcacgc agtgtgcggg cgacgacctt
361 gacgcctcga tcgtgcccgt cgagaccgat cttagcgagc atgatgcgga tcggagcggt
421 gtcgcctgcg tcgatgtcgg agtgtgcgga cagcggcatg gggctcccta ggttggcctg
481 tggcgtgtgc cacat
Sequenz Nr. 14, 164 aa, Nocardioides sp. JS614
   ZP_00656560; (mit Sequenz 4 von HCM-10: Identität = 72/120 (60%), Positives = 94/120 (78%), Gaps = 1/120 (0%))

1 mwhtpqanlg apmplsahsd idagdtapir imlakigldg hdrgvkvvar tlrdagmevv
61 ytglhrspeq vleaavqedv dvlgisllsg ahltifgrlf tliadlpytp rfavvaggvm
121 pdedertlie lgvaavlgqd taprhivevv tdaanqamq veas

## Patentansprüche

1. Verfahren zur enzymatischen Herstellung von 2-Hydroxy-2-methylcarbonsäuren aus 3-Hydroxycarbonsäuren, **dadurch gekennzeichnet, dass** eine 3-Hydroxycarbonsäure in einer wässrigen Reaktionslösung, die eine 3-Hydroxycarbonsäure-CoA-Mutase-Aktivität aufweisende Einheit enthält, welche sowohl 3-Hydroxycarbonyl-CoA-Ester produzierende als auch 3-Hydroxycarbonyl-CoA-Ester isomerisierende Aktivität besitzt, produziert und/oder zu dieser Reaktionslösung gegeben wird, inkubiert und anschließen die entsprechend umgewandelte 2-Hydroxy-2-methylcarbonsäure als Säure oder in Form ihrer Salze gewonnen wird.

2. Verfahren nach Anspruch 1, **dadurch gekennzeichnet, dass** die 3-Hydroxycarbonsäure-CoA-Mutase-Aktivität aufweisende Einheit eine isolierte cobalaminabhängige Mutase umfasst.

3. Verfahren nach Anspruch 1 oder 2, **dadurch gekennzeichnet, dass** die 3-Hydroxycarbonsäure-CoA-Mutase-Aktivität aufweisende Einheit ein 3-Hydroxycarbonyl-CoA-Ester produzierendes Enzym oder Enzymsystem umfasst.

4. Verfahren nach einem der Ansprüche 1 bis 3, **dadurch gekennzeichnet, dass** die 3-Hydroxycarbonsäure-CoA-Mutase-Aktivität aufweisende Einheit ein Mikroorganismus oder ein Rohextrakt davon ist.

5. Verfahren nach Anspruch 1 oder 4, **dadurch gekennzeichnet, dass** man Mikroorganismen, die eine cobalaminabhängige Mutase produzieren bzw. umfassen, 3-Hydroxycarbonsäure-CoA-Mutase-Aktivität besitzen und sowohl 3-Hydroxycarbonyl-CoA-Ester produzierende als auch 3-Hydroxycarbonyl-CoA-Ester isomerisierende Aktivität aufweisen, in wässrigen Systemen zur Umwandlung von 3-Hydroxycarbonsäuren in die entsprechende 2-Hydroxy-2-methylcarbonsäure einsetzt.

6. Verfahren nach einem der Ansprüche 1 bis 5, **dadurch gekennzeichnet, dass**
a) Mikroorganismen mit 3-Hydroxycarbonsäure-CoA-Mutase-Aktivität, die sowohl 3-Hydroxycarbonyl-CoA-Thioester produzierende Aktivität als auch 3-Hydroxycarbonyl-CoA-Thioester isomerisierende Aktivität besitzen in einem wässrigen System mit nachwachsenden Rohstoffen oder aus der Verwertung nachwachsender Rohstoffe anfallender Abfallprodukte als Kohlenstoff- und Energiequelle kultiviert werden, wodurch intrazellulär 3-Hydroxycarbonsäure-CoA-Thioester synthetisiert und zu den korrespondierenden 2-Hydroxy-2-methylcarbonsäuren umgewandelt werden, und
b) die entsprechende 2-Hydroxy-2-methylcarbonsäure als Säure oder in Form ihrer Salze isoliert wird.

7. Verfahren nach einem der Ansprüche 1 bis 6, **dadurch gekennzeichnet, dass** die Reaktion unter Zugabe externer 3-Hydroxycarbonsäure erfolgt.

8. Verfahren nach einem der Ansprüche 1 bis 7, **dadurch gekennzeichnet, dass** der Mikroorganismus ausgewählt aus Bakterienstamm HCM-10 DSM 18028, *Xanthobacter autotrophicus* Py2, *Rhodobacter sphaeroides* (ATCC17029) oder *Nocardioides sp.* JS614 ist.

9. Verfahren nach einem der Ansprüche 1 bis 8, **dadurch gekennzeichnet, dass** ganze Zellen der Mikroorganismen, unverändert, permeabilisiert oder trägerfixiert eingesetzt werden.

10. Verfahren nach einem der Ansprüche 1 bis 8, **dadurch gekennzeichnet, dass** Zellextrakte und/oder die cobalaminabhängige Mutase, und ggf. die weiteren Enzyme, wie z.B. CoA-Ester synthetisierende Enzyme nach teilweiser oder vollständiger Isolierung aus den Mikroorganismen, ggf. in gereinigter Form, eingesetzt werden.

11. Verfahren nach Anspruch 10, **dadurch gekennzeichnet, dass** zellfreie Rohextrakte der Mikroorganismen eingesetzt werden.

12. Verfahren nach einem der Ansprüche 1 bis 11, **dadurch gekennzeichnet, dass** die Proteine mit den Sequenzen SEQ ID NO: 2 und/oder SEQ ID NO: 4, mit den Sequenzen SEQ ID NO: 5 und/oder SEQ ID NO: 6, die Proteine mit den Sequenzen SEQ ID NO: 9 und/oder SEQ ID NO: 10 oder die Proteine mit den Sequenzen SEQ ID NO: 13 und/oder SEQ ID NO: 14 eingesetzt werden, sowie ihre zu mindestens 60% Homologen.

13. Verfahren nach einem der Ansprüche 1 bis 12, **dadurch gekennzeichnet, dass** zur enzymatischen Umsetzung während der Kultivierung ein Zucker und/oder ein Alkohol und/oder eine organische Säure und/oder ein Kohlenwasserstoff oder deren Derivate als Kohlenstoff und Energiequelle eingesetzt wird.

14. Verfahren nach einem der Ansprüche1 bis 13, **dadurch gekennzeichnet, dass** zur Kultivierung ein Substrat mit einem tert. Butylrest als Kohlenstoff und Energiequelle eingesetzt wird.

15. Verfahren nach einem der Ansprüche 1 bis 14, **dadurch gekennzeichnet, dass** zur Kultivierung tert. Butylalkohol als einzige Kohlenstoff- und Energiequelle in einem Basalmedium eingesetzt wird.

16. Verfahren nach einem der Ansprüche 1 bis 15, **dadurch gekennzeichnet, dass** die enzymatische Umsetzung von einem Zucker und/oder einem Alkohol und/oder einer organische Säure und/oder einem Kohlenwasserstoff oder deren Derivaten zur 3-Hydroxycarbonsäure und von der 3-Hydroxycarbonsäure zur 2-Hydroxy-2-methylcarbonsäure in einem einzigen Verfahrensschritt erfolgt.

17. Verfahren nach einem der Ansprüche 1 bis 16, **dadurch gekennzeichnet, dass** unter Zugabe von externer 3-Hydroxybuttersäure die Herstellung von 2-Hydroxyisobuttersäure erfolgt.

18. Verfahren zur Herstellung von C2-C3 ungesättigten Isoalkensäuren, **dadurch gekennzeichnet, dass** eine 2-Hydroxy-2-methylcarbonsäure nach einem Verfahren gemäss einem der Ansprüche 1-17 hergestellt wird und anschliessend dehydratisiert wird.

19. Verfahren zur Herstellung von Methacrylsäure, **dadurch gekennzeichnet, dass** eine 2-Hydroxyisobuttersäure nach einem Verfahren gemäß einem der Ansprüche 1-17 hergestellt wird und anschliessend dehydratisiert wird.

20. Mikroorganismenstamm HCM-10 - DSM 18028.

21. Nukleinsäuremolekül codierend ein Enzym mit der Aktivität einer cobalaminabhängigen Mutase, ausgewählt aus der Gruppe bestehend aus
a) Nukleinsäuremolekülen, die ein Protein codieren, das die unter Seq. Nr. 2 und/oder Seq. Nr. 4 angegebenen Aminosäuresequenzen umfasst ;
b) Nukleinsäuremolekülen, die die unter Seq. Nr. 1 und/oder Seq. Nr. 3 dargestellte Nukleotidsequenz umfassen.

22. Nukleinsäuremolekül nach Anspruch 21, **dadurch gekennzeichnet, dass** es ein Protein codiert, das als oligomeres Enzym zusamrriengesetzt aus zwei verschiedenen Untereinheiten die unter Seq. Nr. 2 und Seq. Nr. 4 angegebenen Aminosäuresequenzen umfasst. ,

23. Nukleinsäuremolekül nach Anspruch 21 oder 22, das ein DNA-Molekül ist.

24. Nukleinsäuremolekül nach Anspruch 23, das eine cDNA oder genomische DNA ist. .

25. Nukleinsäuremolekül nach Anspruch 21 oder 22, das ein RNA-Molekül ist.

26. Protein mit der Aktivität einer cobalaminabhängigen Mutase codiert von einem Nukleinsäuremolekül gemäß Anspruch 21 bis 22.

27. Protein mit der Sequenz Nr. 2.

28. Protein mit der Sequenz Nr. 4, und seine zu mindestens 99% Homologen.

29. Protein als heterodimeres Enzym umfassend Sequenz Nr. 2 und Sequenz Nr. 4.

## Claims

1. Method for the enzymatic production of 2-hydroxy-2-methyl carboxylic acids from 3-hydroxy carboxylic acids, **characterized in that** a 3-hydroxy carboxylic acid is produced in and/or added to an aqueous reaction solution comprising a unit having 3-hydroxy-carboxylate-C o A mutase activity, which has both 3-hydroxy-carbonyl-CoA ester-producing and 3-hydroxy-carbonyl-CoA ester-isomerizing activity, and, after incubation, the correspondingly converted 2-hydroxy-2-methyl carboxylic acid is isolated as acid or in the form of its salts.

2. Method according to Claim 1, **characterized in that** the unit having 3-hydroxy-carboxylate-CoA mutase activity comprises an isolated cobalamin-dependent mutase.

3. Method according to Claim 1 or 2, **characterized in that** the unit having 3-hydroxy-carboxylate-CoA mutase activity comprises a 3-hydroxy-carbonyl-CoA ester-producing enzyme or enzyme system.

4. Method according to any of Claims 1 to 3, **characterized in that** the unit having 3-hydroxy-carboxylate-CoA mutase activity is a microorganism or a crude extract thereof.

5. Method according to Claim 1 or 4, **characterized in that** microorganisms producing or comprising a cobalamin-dependent mutase, having 3-hydroxy-carboxylate-CoA mutase activity and having both 3-hydroxy-carbonyl-CoA ester-producing and 3-hydroxy-carbonyl-CoA ester-isomerizing activity are used in aqueous systems for converting 3-hydroxy carboxylic acids to the corresponding 2-hydroxy-2-methyl carboxylic acid.

6. Method according to any of Claims 1 to 5, **characterized in that**
a) microorganisms with 3-hydroxy-carboxylate-CoA mutase activity, which have both 3-hydroxy-carbonyl-CoA thioester-producing activity and 3-hydroxy-carbonyl-CoA thioester-isomerizing activity, are cultured in an aqueous system with renewable raw materials or waste products deriving from the consumption of renewable raw materials as carbon and energy sources, whereby intracellular 3-hydroxy-carboxylate-CoA thioesters are synthesized and converted to the corresponding 2-hydroxy-2-methyl carboxylic acids, and
b) the corresponding 2-hydroxy-2-methyl carboxylic acid is isolated as acid or in the form of its salts.

7. Method according to any of Claims 1 to 6, **characterized in that** the reaction is carried out with the addition of external 3-hydroxy carboxylic acid.

8. Method according to any of Claims 1 to 7, **characterized in that** the microorganism is selected from bacterial strain HCM-10 DSM 18028, Xanthobacter autotrophicus Py2, Rhodobacter sphaeroides (ATCC17029) or Nocardioides sp. JS614.

9. Method according to any of Claims 1 to 8, **characterized in that** intact cells of the microorganisms are used unchanged, permeabilized or fixed to a support.

10. Method according to any of Claims 1 to 8, **characterized in that** cell extracts and/or the cobalamin-dependent mutase and, where appropriate, the other enzymes such as, for example, CoA ester-synthesizing enzymes, after partial or complete isolation from the microorganisms are used, where appropriate in a purified form.

11. Method according to Claim 10, **characterized in that** cell-free crude extracts of the microorganisms are used.

12. Method according to any of Claims 1 to 11, **characterized in that** the proteins with the sequences SEQ ID NO: 2 and/or SEQ ID NO: 4, with the sequences SEQ ID NO: 5 and/or SEQ ID NO: 6, the proteins with the sequences SEQ ID NO: 9 and/or SEQ ID NO: 10, or the proteins with the sequences SEQ ID NO: 13 and/or SEQ ID NO: 14, are used, and also their at least 60% homologues.

13. Method according to any of Claims 1 to 12, **characterized in that** a sugar and/or an alcohol and/or an organic acid and/or a hydrocarbon or their derivatives are used as carbon and energy sources for enzymatic conversion during culturing.

14. Method according to any of Claims 1 to 13, **characterized in that** a substrate with a tert-butyl radical is used as carbon source and energy source for culturing.

15. Method according to any of Claims 1 to 14, **characterized in that** tert-butyl alcohol is used as sole carbon source and energy source in a basal medium for culturing.

16. Method according to any of Claims 1 to 15, **characterized in that** the enzymatic conversion of a sugar and/or an alcohol and/or an organic acid and/or a hydrocarbon or their derivatives to the 3-hydroxy carboxylic acid and of the 3-hydroxy carboxylic acid to the 2-hydroxy-2-methyl carboxylic acid is carried out in a single method step.

17. Method according to any of Claims 1 to 16, **characterized in that** 2-hydroxy isobutyric acid is produced with the addition of external 3-hydroxy butyric acid.

18. Method for the production of C2-C3-unsaturated isoalkenoic acids, **characterized in that** a 2-hydroxy-2-methyl carboxylic acid is produced according to a method according to any of Claims 1 to 17 and is then dehydrated.

19. Method for the production of methacrylic acid, **characterized in that** a 2-hydroxy isobutyric acid is produced according to a method according to any of Claims 1 to 17 and is then dehydrated.

20. Microorganism strain HCM-10 - DSM 18028.

21. Nucleic acid molecule coding for an enzyme having the activity of a cobalamin-dependent mutase, selected from the group consisting of
a) nucleic acid molecules coding for a protein comprising the amino acid sequences indicated under Seq. No. 2 and/or Seq. No. 4;
b) nucleic acid molecules comprising the nucleotide sequence depicted under Seq. No. 1 and/or Seq. No. 3.

22. Nucleic acid molecule according to Claim 21, **characterized in that** it codes for a protein which, as an oligomeric enzyme composed of two different subunits, comprises the amino acid sequences indicated under Seq. No. 2 and Seq. No. 4.

23. Nucleic acid molecule according to Claim 21 or 22, which is a DNA molecule.

24. Nucleic acid molecule according to Claim 23, which is a cDNA or genomic DNA.

25. Nucleic acid molecule according to Claim 21 or 22, which is an RNA molecule.

26. Protein having the activity of a cobalamin-dependent mutase encoded by a nucleic acid molecule according to Claims 21 to 22.

27. Protein with the sequence No. 2.

28. Protein with the sequence No. 4 and its at least 99% homologues.

29. Protein as heterodimeric enzyme comprising sequence No. 2 and sequence No. 4.

## Revendications

1. Procédé pour la préparation enzymatique d'acides 2-hydroxy-2-méthylcarboxyliques à partir d'acides 3-hydroxycarboxyliques, **caractérisé en ce qu'**on produit un acide 3-hydroxycarboxylique dans une solution réactionnelle aqueuse, qui contient une unité présentant une activité d'acide 3-hydroxycarboxylique-CoA-mutase, qui possède une activité de production de 3-hydroxycarbonyl-CoA-ester ainsi que d'isomérisation de 3-hydroxycarbonyl-CoA-ester et/ou on ajoute cet acide 3-hydroxycarboxylique à cette solution réactionnelle, on incube puis on génère l'acide 2-hydroxy-2-méthylcarboxylique converti de manière correspondante sous forme d'acide ou sous forme de ses sels.

2. Procédé selon la revendication 1, **caractérisé en ce que** l'unité présentant une activité d'acide 3-hydroxycarboxylique-CoA-mutase comprend une mutase isolée dépendant de la cobalamine.

3. Procédé selon la revendication 1 ou 2, **caractérisé en ce que** l'unité présentant une activité d'acide 3-hydroxycarboxylique-CoA-mutase comprend une enzyme ou un système enzymatique produisant un 3-hydroxycarbonyl-CoA-ester.

4. Procédé selon l'une quelconque des revendications 1 à 3, **caractérisé en ce que** l'unité présentant une activité d'acide 3-hydroxycarboxylique-CoA-mutase est un microorganisme ou un extrait brut de celui-ci.

5. Procédé selon la revendication 1 ou 4, **caractérisé en ce qu'**on utilise des microorganismes qui produisent ou comprennent une mutase dépendant de la cobalamine, qui possèdent une activité d'acide 3-hydroxycarboxylique-CoA-mutase et qui présentent une activité de production de 3-hydroxycarbonyl-CoA-ester ainsi que d'isomérisation de 3-hydroxycarbonyl-CoA-ester, dans des systèmes aqueux pour la conversion d'acides 3-hydroxycarboxyliques en acides 2-hydroxy-2-méthylcarboxyliques correspondants.

6. Procédé selon l'une quelconque des revendications 1 à 5, **caractérisé en ce que**
a) des microorganismes présentant une activité d'acide 3-hydroxycarboxylique-CoA-mutase, qui possèdent une activité de production de 3-hydroxycarbonyl-CoA-thioester ainsi qu'une activité d'isomérisation de 3-hydroxycarbonyl-CoA-thioester sont cultivés dans un système aqueux avec des matières premières renouvelables ou des déchets obtenus de la valorisation de matières premières renouvelables comme source de carbone et d'énergie, suite à quoi des CoA-thioesters de l'acide 3-hydroxycarboxylique sont synthétisés et transformés en acides 2-hydroxy-2-méthylcarboxyliques correspondants, et
b) l'acide 2-hydroxy-2-méthylcarboxylique correspondant est isolé sous forme d'acide ou sous forme de ses sels.

7. Procédé selon l'une quelconque des revendications 1 à 6, **caractérisé en ce que** la réaction est réalisée par addition d'acide 3-hydroxycarboxylique externe.

8. Procédé selon l'une quelconque des revendications 1 à 7, **caractérisé en ce que** le microorganisme est choisi parmi les souches bactériennes HCM-10 DSM 18028, Xanthobacter autotrophicus Py2, Rhodobacter sphaeroides (ATCC17029) ou Nocardioides sp. JS614.

9. Procédé selon l'une quelconque des revendications 1 à 8, **caractérisé en ce qu'**on utilise des cellules entières des microorganismes, non modifiées, perméabilisées ou fixées sur un support.

10. Procédé selon l'une quelconque des revendications 1 à 8, **caractérisé en ce qu'**on utilise des extraits cellulaires et/ou la mutase dépendant de la cobalamine, et le cas échéant les autres enzymes, comme par exemple les enzymes synthétisant le CoA-Ester après un isolement partiel ou complet des microorganismes, le cas échéant sous forme purifiée.

11. Procédé selon la revendication 10, **caractérisé en ce qu'**on utilise des extraits bruts exempts de cellules des microorganismes.

12. Procédé selon l'une quelconque des revendications 1 à 11, **caractérisé en ce qu'**on utilise les protéines présentant les séquences SEQ ID NO : 2 et/ou SEQ ID NO : 4, présentant les séquences SEQ ID NO : 5 et/ou SEQ ID NO : 6, les protéines présentant les séquences SEQ ID NO : 9 et/ou SEQ ID NO : 10 ou les protéines présentant les séquences SEQ ID NO : 13 et/ou SEQ ID NO : 14, ainsi que leurs homologues présentant une homologie d'au moins 60%.

13. Procédé selon l'une quelconque des revendications 1 à 12, **caractérisé en ce qu'**on utilise, pour la transformation enzymatique, pendant la culture, un sucre et/ou un alcool et/ou un acide organique et/ou un hydrocarbure ou leurs dérivés comme source de carbone et d'énergie.

14. Procédé selon l'une quelconque des revendications 1 à 13, **caractérisé en ce qu'**on utilise pour la culture un substrat présentant un radical tert-butyle comme source de carbone et d'énergie.

15. Procédé selon l'une quelconque des revendications 1 à 14, **caractérisé en ce qu'**on utilise pour la culture de l'alcool tert-butylique comme source unique de carbone et d'énergie dans un milieu de base.

16. Procédé selon l'une quelconque des revendications 1 à 15, **caractérisé en ce que** la transformation enzymatique d'un sucre et/ou d'un alcool et/ou d'un acide organique et/ou d'un hydrocarbure ou de leurs dérivés en acide 3-hydroxycarboxylique et de l'acide 3-hydroxycarboxylique en acide 2-hydroxy-2-méthylcarboxylique est réalisée en une seule étape de procédé.

17. Procédé selon l'une quelconque des revendications 1 à 16, **caractérisé en ce que** la préparation de l'acide 2-hydroxyisobutyrique est réalisée avec addition d'acide 3-hydroxybutyrique externe.

18. Procédé pour la préparation d'acides isoalcéniques insaturés en C2-C3, **caractérisé en ce qu'**un acide 2-hydroxy-2-méthylcarboxylique est préparé selon un procédé selon l'une quelconque des revendications 1-17, puis est déshydrogéné.

19. Procédé pour la préparation d'acide méthacrylique, **caractérisé en ce qu'**un acide 2-hydroxyisobutyrique est préparé selon un procédé selon l'une quelconque des revendications 1-17, puis est déshydrogéné.

20. Souche de microorganismes HCM-10-DSM 18028.

21. Molécule d'acide nucléique codant pour une enzyme présentant l'activité d'une mutase dépendant de la cobalamine, choisie dans le groupe constitué par
a) les molécules d'acide nucléique qui codent pour une protéine qui comprend les séquences d'acides aminés indiquées dans Seq. n° 2 et/ou Seq. n° 4 ;
b) les molécules d'acide nucléique qui comprennent la séquence nucléotidique représentée dans Seq. n° 1 et/ou Seq. n° 3.

22. Molécule d'acide nucléique selon la revendication 21, **caractérisée en ce qu'**elle code pour une protéine qui comprend comme enzyme oligomère, composée par deux sous-unités différentes, les séquences d'acides aminés indiquées dans Seq. n° 2 et/ou Seq. n° 4.

23. Molécule d'acide nucléique selon la revendication 21 ou 22, qui est une molécule d'ADN.

24. Molécule d'acide nucléique selon la revendication 23, qui est de l'ADNc ou de l'ADN génomique.

25. Molécule d'acide nucléique selon la revendication 21 ou 22, qui est une molécule d'ARN.

26. Protéine présentant l'activité d'une mutase dépendant de la cobalamine, codée par une molécule d'acide nucléique selon la revendication 21 à 22.

27. Protéine présentant la séquence n° 2.

28. Procédé présentant la séquence n° 4 et ses homologues présentant une homologie d'au moins 99%.

29. Protéine comme enzyme hétérodimère comprenant la séquence n° 2 et la séquence n° 4.
